# EUROPEAN PATENT APPLICATION

(11) **EP 3 967 357 A1**
(43) Date of publication of application: **16.03.2022**
(21) Application number: 21193203.3
(22) Date of filing: 26.08.2021
(51) Int. Cl.: A61M 25/10, A61M 25/00, A61B 17/22

(54) **IMPROVED FLEXIBILITY BALLOON CATHETER**

(30) Priority: 28.08.2020 US 202017006137
(71) Applicant: DePuy Synthes Products, Inc., Raynham, MA 02767 (US)
(72) Inventor: LORENZO, Juan A., New Hope, 18938 (US); JOHNSON, Kirk, Raynham, 02767 (US)
(74) Representative: Carpmaels & Ransford LLP

(57) **Abstract**

A balloon guide catheter 100a having a substantially tubular body 115, an inflation lumen 117, and a balloon 113. The tubular body can include an inner hollow lumen 114 and a distal end. The inflation lumen can have a distal end and a proximal section. The distal end of the inflation lumen can be in connection with the tubular body approximate to the distal end of the tubular body. The proximal section of the inflation lumen can extend a majority of the length of the tubular body and can be moveable in relation to the tubular body. The inflation lumen can be positioned within an inner hollow lumen of a tubular body of the catheter or external to the tubular body, thereby enhancing flexibility of the catheter.

## Description

### FIELD OF INVENTION

The present invention relates generally to devices and methods used in removing acute blockages from blood vessels during intravascular medical treatments. More specifically, the present invention relates to balloon guide catheters used to occlude portions of target blood vessels during such procedures.

### BACKGROUND

Balloon guide catheters can be used in standard arterial or in ischemic stroke procedures to act as a conduit for diagnostic and therapeutic devices while also providing flow arrest, flow control, and/or flow reversal to aid in the safe retrieval of a clot from the patient. This flow control provides distal vascular protection which is important to minimize the risk of emboli migration and other procedural complications, particularly in the small and fragile cerebral passages for stroke patients. Additionally, proximal flow control provided by these catheters has been shown to correlate with better angiographic and clinical outcomes. These catheters can be useful for reducing procedure times and effort during the recanalization process of intra-arterial mechanical thrombectomy procedures. They can limit the number of clot retrieval attempts needed during ischemic stroke, for example, while also reducing the occurrence of distal emboli.

A balloon guide catheter must be sufficiently flexible while also having the axial stiffness to be delivered smoothly through tortuous vasculature to the target site (typically the internal carotid artery and on into the cerebral vasculature for ischemic stroke patients). Designing the balloon guide catheter to have proper flexibility can be complicated by the need to include an inflation lumen to inflate the balloon of the balloon guide catheter.

The devices must also be designed to be as atraumatic as possible while still delivering a high level of performance. Once access to the target is gained and the balloon deployed, the guide catheter has to be appreciably robust to remain stable in that position while other devices are advanced, manipulated and withdrawn through the lumen. The lumen itself must be of a large enough diameter for these devices while also directing more efficient aspiration necessary to remove blood and thrombus material during the procedure. However, the lumen size must be balanced by the need for an outer diameter as low profile as possible to minimize the size of the entry orifice that must be closed once the catheters are removed from the patient. Designing the balloon guide catheter to have an optimally sized lumen can also be complicated by the need to include an inflation lumen to inflate the balloon of the balloon guide catheter.

### SUMMARY

It is an object of the present invention to provide a balloon guide catheter having a small diameter inflation lumen tube in addition to the central lumen of the catheter. The inflation lumen tube can be positioned within the central lumen of the catheter or external to the body of the catheter such that the inflation lumen tube can inflate the balloon independently of the central lumen.

An example balloon guide catheter can include a substantially tubular body, an inflation lumen, and a balloon. The tubular body can include an inner hollow lumen and a tubular body distal end. The inflation lumen can include an inflation lumen distal end in connection with the tubular body approximate the tubular body distal end and a proximal section extending a majority of the length of the tubular body. The proximal section can be moveable in relation to the tubular body. The balloon can be in fluid communication with the inflation lumen distal end.

The inflation lumen can have an inflation diameter less than a lumen diameter of the inner hollow lumen.

The balloon can be affixed approximate the tubular body distal end.

The tubular body distal end can have a wall thickness that tapers from a wider thickness at a first position to a narrower thickness at a second position. The first position can be in a proximal direction from the balloon and the second position can be under the balloon.

The tubular body distal end can include a flexible portion disposed under the balloon. The flexible portion can be more flexible than a portion of the tubular body approximate the balloon and in a proximal direction from the balloon.

The inflation lumen can be located in the inner hollow lumen. When the inflation lumen is located in the inner hollow lumen, the inflation diameter can be less than a lumen diameter of the inner hollow lumen. The inflation lumen can be at least partially eccentric to the tubular body.

The inflation lumen can be located external to the tubular body.

The disclosed technology also includes a catheter including a substantially tubular body having an inner hollow lumen, an inner surface defining the inner hollow lumen extending therethrough, a fluidic lumen extending a majority of the length of the tubular body, and an outer sleeve surrounding the tubular body and fluidic lumen. The outer sleeve can extend a majority of the length of the tubular body. The outer sleeve can be sufficient to secure the fluidic lumen to the tubular body along the majority of the length of the tubular body.

The tubular body can include an outer surface and the fluidic lumen can be disposed on the outer surface of the tubular body.

The fluidic lumen can be disposed on a side of the outer surface.

The catheter can further include a balloon affixed to a distal end of the tubular body. The balloon can be in fluid communication with the fluidic lumen.

The fluidic lumen can have a substantially crescent-shape cross-section.

The fluidic lumen can include a polymer such as PET.

The tubular body can include at least one reinforcing braid or coil.

The disclosed technology can also include a method for deploying a balloon guide catheter. The method can include advancing a balloon guide catheter into a patient's vasculature. The balloon guide catheter can include a tubular body with a distal end, an inner hollow lumen, an inflation lumen with a distal end affixed to the tubular body approximate the distal end of the tubular body and extending a majority of the length of the tubular body, and a balloon. The method can include flexing the inflation lumen independent of the tubular body as the catheter is advanced through the vasculature to facilitate flexible movement of the catheter and inflating the balloon through the inflation lumen.

The method can further include advancing an intermediate catheter through the balloon guide catheter to the target location and aspirating an occlusive thrombus.

The method can further include advancing a microcatheter and clot retrieval device through the balloon guide catheter to the target location and deploying the clot retrieval device to capture an occlusive thrombus and retrieve the thrombus with aspiration.

The method can further include advancing an intermediate catheter, microcatheter, and clot retrieval device through the balloon guide catheter to the target location and deploying the clot retrieval device to capture an occlusive thrombus and retrieve the thrombus with aspiration.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1 is an illustration of a balloon guide catheter according to aspects of the present invention;
FIG. 2A is a view of a catheter tip of the balloon guide catheter illustrated in FIG. 1 according to aspects of the present invention;
FIG. 2B is a side cross-section view of the catheter tip of balloon guide catheter illustrated in FIG. 2A according to aspects of the present invention;
FIG 3 is an illustration of another example balloon guide catheter having an external inflation lumen according to aspects of the present invention;
FIG. 4A is a view of the catheter tip of the balloon guide catheter illustrated in FIG. 3 according to aspects of the present invention;
FIG. 4B is perspective view of the catheter tip of the balloon guide catheter having an external inflation lumen according to aspects of the present invention;
FIG. 5A is a cross-section view of another balloon guide catheter having a fluidic lumen in a deflated state according to aspects of the present invention;
FIG. 5B is a perspective view of the balloon guide catheter illustrated in FIG. 5A with a balloon in an inflated state according to aspects of the present invention;
FIG. 5C is a cross-section view of the balloon guide catheter illustrated in FIG. 5B according to aspects of the present invention;
FIG. 6A illustrates the balloon guide catheter being used with an intermediate catheter, microcatheter, and clot retrieval device to capture a target occlusion in a blood vessel according to aspects of the present invention;
FIG. 6B illustrates the balloon guide catheter with a microcatheter and clot retrieval device according to aspects of the present invention; and
FIG. 7 is a flow diagram outlining steps for deploying a balloon guide catheter according to aspects of the present invention.

### DETAILED DESCRIPTION

A balloon guide catheter must be sufficiently flexible while also having the axial stiffness to be delivered smoothly through tortuous vasculature to the target sites. In order to achieve the desired flexibility, an inflation lumen having a small diameter as compared to a central lumen of the balloon guide catheter can be provided. The inflation lumen can be positioned within the central lumen of a tubular body of the catheter. Alternatively, the inflation lumen can be positioned external to the tubular body. By removing the inflation lumen from within an outer layer of the tubular body, the flexibility of the balloon guide catheter can be improved, allowing for effective delivery to a target site.

Although example embodiments of the disclosed technology are explained in detail herein, it is to be understood that other embodiments are contemplated. Accordingly, it is not intended that the disclosed technology be limited in its scope to the details of construction and arrangement of components set forth in the following description or illustrated in the drawings. The disclosed technology is capable of other embodiments and of being practiced or carried out in various ways.

It must also be noted that, as used in the specification and the appended claims, the singular forms "a," "an" and "the" include plural referents unless the context clearly dictates otherwise. By "comprising" or "containing" or "including" it is meant that at least the named compound, element, particle, or method step is present in the composition or article or method, but does not exclude the presence of other compounds, materials, particles, method steps, even if the other such compounds, material, particles, method steps have the same function as what is named.

In describing example embodiments, terminology will be resorted to for the sake of clarity. It is intended that each term contemplates its broadest meaning as understood by those skilled in the art and includes all technical equivalents that operate in a similar manner to accomplish a similar purpose. It is also to be understood that the mention of one or more steps of a method does not preclude the presence of additional method steps or intervening method steps between those steps expressly identified. Steps of a method may be performed in a different order than those described herein without departing from the scope of the disclosed technology. Similarly, it is also to be understood that the mention of one or more components in a device or system does not preclude the presence of additional components or intervening components between those components expressly identified.

As discussed herein, vasculature can be that of any "subject" or "patient" including of any human or animal. It should be appreciated that an animal may be a variety of any applicable type, including, but not limited thereto, mammal, veterinarian animal, livestock animal or pet type animal, etc. As an example, the animal may be a laboratory animal specifically selected to have certain characteristics similar to a human (e.g., rat, dog, pig, monkey, or the like). It should be appreciated that the subject may be any applicable human patient, for example.

As discussed herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

The figures illustrate a generally hollow or tubular structure according to the present invention. When used herein, the terms "tubular" and "tube" are to be construed broadly and are not limited to a structure that is a right cylinder or strictly circumferential in cross-section or of a uniform cross-section throughout its length. For example, the tubular structure or system is generally illustrated as a substantially right cylindrical structure. However, the tubular system may have a tapered or curved outer surface without departing from the scope of the present invention.

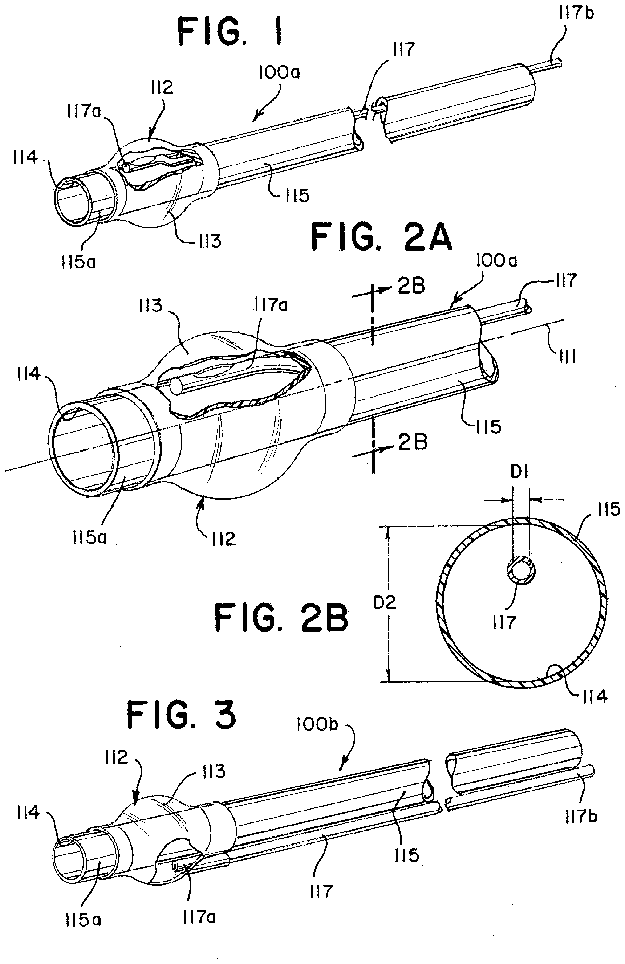

Referring now to the figures, FIGs. 1 and 2A illustrate an example balloon guide catheter 100a. The balloon guide catheter 100a can include a catheter tip 112, a substantially tubular body 115, an inflation lumen 117, and a balloon 113. The tubular body 115 is illustrated as transparent for the purposes of illustration but need not be transparent in practice.

The tubular body 115 can be a thin, elongate, hollow tube. The tubular body 115 can include a hypotube. Commonly used hypotube materials include Nitinol and stainless-steel alloys. The tubular body 115 can have a uniform stiffness across its length, or a stiffness that varies along the length. Variations in the stiffness profile of the tubular body 115 can be created by laser cutting features such as circumferential slots grooves and/or longitudinally or axially offset patterned ridges or recesses. Alternatively, the tubular body 115 can be a polymeric tube. In some instances, an outer surface of the polymeric tube can include ridges and/or recesses to provide enhanced torque, push, and trackability characteristics. Variations in the stiffness profile of the tubular body 115 can be achieved by utilizing layers of polymeric material having different length and stiffness along the tubular body 115.

Both an inner and outer surface of the tubular body 115 can be made from, or coated with, a lubricious low-friction material such as PTFE. This can facilitate navigation of the balloon guide catheter 100a through the vasculature, there is a smooth, non-abrasive surface for contacting the interior vessel walls along the route to the target site.

The tubular body 115 can include an inner hollow lumen 114. The inner hollow lumen 114 can be used for the delivery of other catheters and auxiliary devices to a target site. In some examples, the inner hollow lumen 114 can also provide a channel for aspiration and the injection of contrast. The tubular body 115 can have relatively thin walls such that the inner hollow lumen 114 can have a large cross-sectional area for aspiration and passage of other devices. The size of the inner hollow lumen 114 can vary based on the target site, or a standard guide catheter inner diameter of approximately 0.087" can be used. As illustrated in FIG. 2A, the inner hollow lumen 114 can define a longitudinal axis 111.

The inflation lumen 117 can be substantially tubular and can be disposed within the inner hollow lumen 114. The inflation lumen 117 can have a distal end 117a and a proximal section 117b. The proximal section 117b can extend through the inner hollow lumen 114 along a majority of the length of the tubular body 115. In some embodiments, the proximal section 117b can extend through the inner hollow lumen 114 along the entire length of the tubular body 115. As illustrated in FIG. 2A, the proximal section 117b of the inflation lumen 117 can run substantially parallel with the longitudinal axis 111 such that the inflation lumen 117 is at least partially eccentric with the tubular body 115. Alternatively, the inflation lumen 117 can be disposed approximate to an inner wall of the tubular body 115 such that the inflation lumen 117 is off set from the longitudinal axis 111. At least a portion of the inflation lumen 117 can be moveable in relation to the tubular body 115, thereby providing increased flexibility of the balloon guide catheter 100a. In some examples, a majority of the link of the inflation lumen 117 can be movable in relation to the tubular body. For instance, when the balloon guide catheter 100a is being maneuvered through vasculature, some or all of the length of the inflation lumen 117 within the inner hollow lumen 114 can move in alignment with the tubular body 115. Additionally, or alternatively, some or all of the length of the inflation lumen 117 can move independently from the tubular body 115.

In many treatments, it is desirable for a distal length of the balloon guide catheter 100a to have greater flexibility to navigate vasculature while a proximal length of the balloon guide catheter 100a has greater stiffness to be pushed. In some examples, therefore, the inflation lumen 117 can be movable in relation to the tubular body 115 throughout a majority of the distal length and affixed to the tubular body 115 throughout a majority of the proximal length such that the inner hollow lumen 114 moves in alignment with the inner hollow lumen 114 in the proximal length.

As illustrated in FIG. 2A, the inflation lumen 117 can be disposed within the inner hollow lumen 114 such that the distal end 117a of the inflation lumen 117 terminates proximate the distal end 115a of the tubular body 115. In this configuration, the distal end 117a of the inflation lumen 117 and the balloon 113 can be in fluid communication. The inflation lumen 117 can inject fluid and/or other inflation media to inflate the balloon 113. When inflated the balloon 113 can appose the inner walls of a blood vessel. The inflated balloon 113 can have an ovular or tapered shape with an atraumatic, large, flared radius of curvature for interfacing with the vasculature walls.

The balloon 113 can be mounted approximate to the distal end 115a of the tubular body 115. In some embodiments, the balloon 113 can be mounted to the outer surface of the distal end 115a of the tubular body 115 such that the balloon 113 and the tubular body 115 are flush with one another. Because the inflation lumen 117 is entirely within the inner hollow lumen 114, the balloon 113 can be mounted flush with the outer surface of the distal end 115a of the tubular body 115 along the entire circumference of the tubular body 115. Flush mounting can provide a smooth delivery profile for the balloon guide catheter 100a. The balloon 113 can be mounted to the outer surface of the distal end 115a by a mechanical member, such as a ring-shaped tie band or strap, or it could be crimped, welded, or attached by other means. In one example, the balloon is attached using suitable adhesive means, such as epoxy or cyanacrylate.

The distal end 115a of the tubular body 115 can be modified in order to increase the flexibility of the balloon guide catheter 100a. By way of example, the walls of the distal end 115a can have a reduced thickness as compared to the walls of the tubular body 115 extending from the distal end 115a. In this sense, the tubular body 115 can have a wall thickness that tapers from a wider thickness at a first position to a narrower thickness at a second position. The first position can be proximal in relation to the balloon 113. The second position can be under the balloon 113. Alternatively or in addition to, a flexible material can be disposed at the distal end 115a of the tubular body 115 such that the flexible material is disposed under the balloon 113. By way of example, the flexible material can include elastomeric material, including but not limited to, Pebax ^{®} elastomers having durometers of between approximately 25D and approximately 40D and blends of Pebax^{®} elastomers within the same range of durometers. The flexible material can be a different material than the tubular body 115. The flexible material can be more flexible than the material of the tubular body 115. These modifications to the distal end 115a of the tubular body 115 can increase flexibility of the balloon guide catheter 100a at the distal end 115a, while also maintaining axial stiffness along a majority of the length of the catheter 100a such that the balloon guide catheter 100a can safely and effectively maneuver through the vasculature of a patient to a target site.

The balloon 113 can be constructed of any of a number of materials, such as chloroprene, polyurethane, nylon, PBx, or any other thermoplastic elastomer. These materials allow the balloon 113 to be durable and thin. The balloon 113 can be permeable to gas. Alternatively, the balloon 113 can be impermeable to gas. The outer surface of the balloon 113 can be coated with a hydrophilic coating for atraumatic lubricity, and the balloon 113 can be shaped such that there is minimal contract with the vessel wall when inflated.

FIG. 2B illustrates a side cross-section view of the catheter tip 112 of the balloon guide catheter 100a illustrated in FIG. 2A. The tubular body 115 can include an inner hollow lumen 114. The inflation lumen 117 can be disposed within the inner hollow lumen 114. In some instances, the inflation lumen 117 is disposed within the inner hollow lumen 114 and approximate to a top inner surface of the tubular body 115, as illustrated in FIG. 2B. In some instances, the inflation lumen 117 can be disposed within the inner hollow lumen 114 and approximate to a side inner surface or a bottom inner surface of the tubular body 115. In some instances, the inflation lumen 117 can be disposed substantially eccentric to the inner hollow lumen 114 and the tubular body 115. The illustrated portion of the inflation lumen 117 is free to move in relation to the inner hollow lumen 114 as the catheter 100a is bent.

The inflation lumen 117 can have a first diameter D1 and the inner hollow lumen 114 can have a second diameter D2. The first diameter D1 can be less than the second diameter D2. In some embodiments, the first diameter D1 can be approximately a quarter of the second diameter D2. In some embodiments, the first diameter D1 can be approximately half of the second diameter D2. The first diameter D1 can be less than the second diameter D2 because the volume of inflation media necessary to inflate the balloon is typically not substantial.

FIGs. 3 through 4B illustrate the balloon guide catheter 100b having an external inflation lumen 117 with respect to the tubular body 115. The inflation lumen 117 can have a distal end 117a and a proximal section 117b. The proximal section 117b can extend from the distal end 117a and can extend a majority of the length of the tubular body 115. The distal end 117a can be approximate the distal end 115a of the tubular body 115. The balloon 113 can be joined to at least a portion of the outer surface of the distal end 115a of the tubular body 115 and at least a portion of the distal end 117a of the inflation lumen 117 such that the inflation lumen 117 can be in fluid communication with the balloon 113. The balloon 113 can be joined to the outer surface of the tubular body 115 and the outer surface of the distal end 117a of the inflation lumen 117 using any attachment mechanism, including but not limited to, a ring-shaped tie band or strap, adhesives, crimping, or welding.

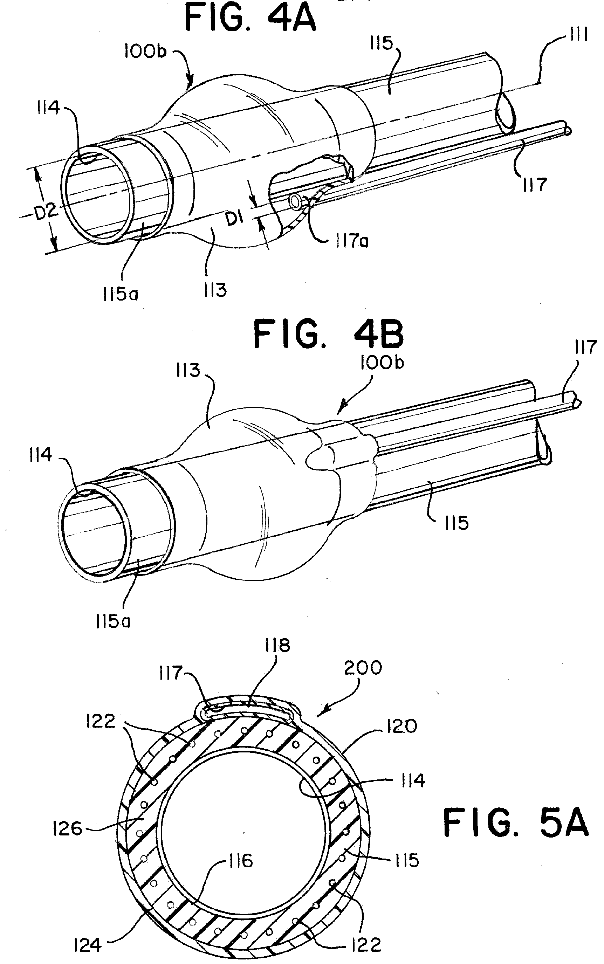

As illustrated in FIGs. 4A, the inflation lumen 117 can run parallel to the tubular body 115 with respect to the longitudinal axis 111. The inflation lumen 117 is illustrated as being radially downwards, or "6 o'clock" orientation with respect to the tubular body 115 and inner hollow lumen 114. Although the exterior surface of the inflation lumen 117 and the exterior surface of the tubular body 115 can contact each other as the inflation lumen 117 runs parallel to the tubular body 115, over some or all of the length of the inflation lumen 117, the exterior surface of the inflation lumen 117 is not affixed to the exterior surface of the tubular body 115 such that the inflation lumen 117 can be moved and/or manipulated dependently or independently of the tubular body 115 over the unaffixed length(s). Configured as such, as the balloon guide catheter 100b is maneuvered through vasculature, where the inflation lumen 117 is unaffixed to the tubular body 115, the catheter 100b can have increased flexibility compared to the flexibility of the catheter 100b were the inflation lumen 117 affixed to the tubular body 115. This increased flexibility can facilitate effective delivery of the balloon guide catheter 100b to a target location within a blood vessel.

FIG. 4B illustrates the balloon guide catheter 100b having an external inflation lumen 117. The inflation lumen 117 is illustrated as being radially upwards, or approximately at a "12 o'clock" orientation with respect to the tubular body 115 and the inner hollow lumen 114. The balloon 113 can be joined to at least a portion of the outer surface of the distal end 115a of the tubular body 115 and at least a portion of the distal end 117a of the inflation lumen 117 such that the inflation lumen 117 can be in fluid communication with the balloon 113. The balloon 113 can be joined to the outer surface of the distal end 115a of the tubular body 115 and the outer surface of the distal end 117a of the inflation lumen 117 using any attachment mechanism, including but not limited to, a ring-shaped tie band or strap, adhesives, crimping, or welding.

Because the first diameter D1 of the inflation lumen 117 can be relatively small, the inflation lumen 117 does not substantially affect the exterior profile of the tubular body 115. In this sense, the balloon guide catheter 100b can be effectively maneuvered through the vasculature.

By positioning the inflation lumen 117 either within the inner hollow lumen 114 or directly along the exterior surface of the tubular body 115 as illustrated in FIGs. 1 through 4B, the balloon guide catheter 100 can exhibit improved flexibility from other prior balloon guide catheters that including an inflation lumen within an outer layer of the tubular body. The improved flexibility can facilitate safe and effective navigation and positioning of the balloon guide catheter 100 within the vasculature.

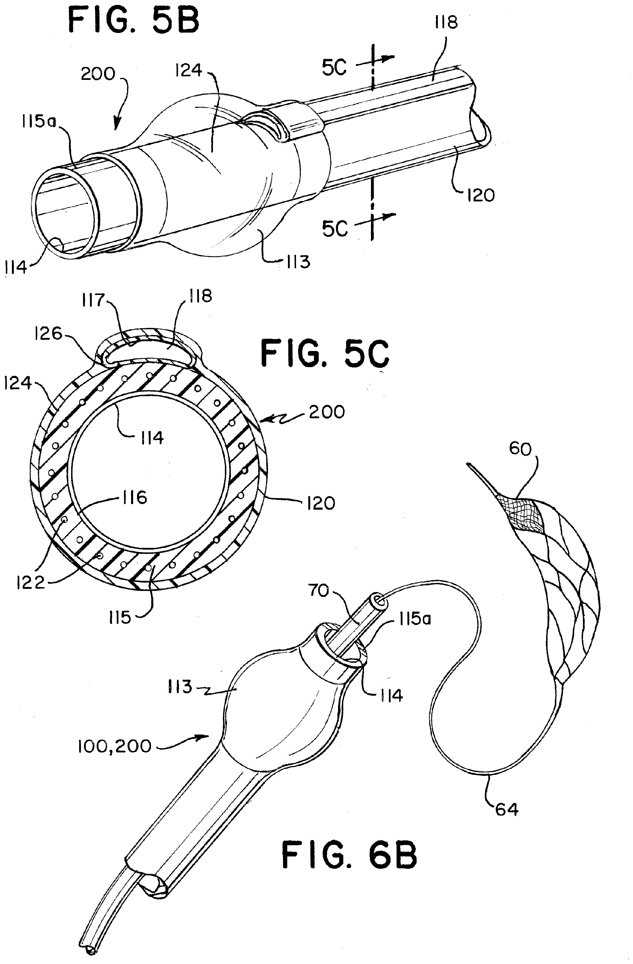

FIGs 5A-5C illustrates a balloon guide catheter 200 having a fluidic lumen 118. FIG. 5A illustrates a cross-section view of the balloon guide catheter 200 in a deflated state, where the cross-section is taken in a proximal direction in relation to the balloon 113 as a similar location as the cross-section for FIG. 5C as indicated in FIG. 5B. The balloon guide catheter 200 can include a substantially tubular body 115. The tubular body 115 can have an inner surface 116 and an outer surface 124. The inner surface 116 can define the inner hollow lumen 114 and can extend the length of the tubular body 115. The inner hollow lumen 114 can be used for the delivery of other catheters and auxiliary devices to a target site and also as a channel for aspiration and injection of contrast.

The balloon guide catheter 200 can include a fluidic lumen 118 disposed on a side 126 of the outer surface 124 of the tubular body 115. The fluidic lumen 118 can extend a majority of the length of the tubular body 115. In some embodiments, the fluidic lumen 118 can extend the entire length of the tubular body 115.

The fluidic lumen 118 can have a variety of cross-section shapes. By way of example, the fluidic lumen 118 can have a substantially crescent-shaped cross-section, as illustrated in FIGs. 5A through 5C. Alternatively, the fluidic lumen 118 can have a substantially, circular, ellipsoid, or triangular, or polygonal shaped cross-section.

The fluidic lumen 118 can be substantially made of thin, non-complaint material. The fluidic lumen 118 can be made substantially of one or more thermoplastic polymers, including but not limited to polyethylene terephthalate (PET), polytetrafluoroethylene (PTFE), polyethylene (PE), fluorinated ethylene propylene (FEP), and the like.

An outer sleeve 120 can surround the outer surface 124 of the tubular body 115 and the fluidic lumen 118. The outer sleeve 120 can extend a majority of the length of the tubular body 115. In some embodiments, the outer sleeve 120 can extend the entire length of the tubular body 115. The outer sleeve 120 can provide structural stability and can secure the fluidic lumen 118 to the tubular body 115 along the majority of the length of the tubular body 115. The outer sleeve 120 can be substantially made of an elastic material and can provide outer surface lubricity without significant increase in stiffness due to the softness of the elastic material. By way of example, the elastic material can be high density polyethylene.

Advancing through particularly tortuous vasculature can cause the balloon guide catheter 200 and the inflation lumen 117 and/or the inner hollow lumen 114 to kink or crimp. Kinking of the inner hollow lumen 114 can lead to binding on a guidewire or other devices. A kinked inflation lumen 117 can inhibit flow to and from the balloon 113 of the balloon guide catheter 200. In some extreme cases, a kinked inflation lumen 117 can result in the complete failure of the balloon or loss of the ability to inflate or deflate the balloon 113. This can create complications during a treatment as the non-functioning balloon guide catheter may need to be removed, a physician may need to conduct a procedure without blood-flow arrest, or some other device may need to be inserted through the inflation lumen to deflate the balloon.

To minimize kinking, the tubular body 115 can include a support structure 122. The support structure 122 can be disposed between the inner surface 116 of the tubular body 115 and the outer surface 124 of the tubular body 115. The support structure 122 can be at least one reinforcing element. The reinforcing element can be a reinforcing braid and/or wire coil. The support structure 122 can include any number of reinforcing elements. The density of the braid and/or coils or the materials used in their construction can be adjusted and manipulated to vary the axial stiffness profile of the balloon guide catheter 200. For example, changing the density, material, or configuration of the braid and/or coils can increase flexibility of the distal end 115a of the tubular body 115 while still minimizing the likelihood of kinking of the inflation lumen 117.

FIGs. 5B and 5C illustrate the balloon guide catheter 200 in an inflated state. The balloon 113 can be joined to the outer surface 124 of the distal end 115a of the tubular body 115 and the outer sleeve 120 such that the fluidic lumen 118 is in fluid communication with the balloon 113. Because the balloon is joined to the tubular body 115 at the distal most end and the outer sleeve at the other end, the balloon 113 is not mounted flush with the tubular body 115.

The fluidic lumen 118 can be used as a conduit to inject inflation media to inflate the balloon 113. As illustrated in FIGs. 5A-5C, the fluidic lumen 118 is separate and distinct from the inner hollow lumen 114, such that the fluidic lumen 118 can inflate the balloon 113 independently of the inner hollow lumen 114. FIG. 5C illustrates a cross-section view of the balloon guide catheter 200 in an inflated state. As illustrated in FIG. 5C, the fluidic lumen 118 can expand when the fluidic lumen 118 injects inflation media. Comparing FIG. 5A to FIG. 5C, the fluidic lumen 118 can have a larger cross-sectional area when inflated (FIG. 5C) compared to uninflated (FIG. 5A). The outer sleeve 120 can therefore be configured to allow the fluidic lumen 118 to change in shape when the fluidic lumen 118 is inflated. The outer sleeve 120 can further allow the fluidic lumen 118 to change in shape when the catheter 200 is bent and otherwise manipulated.

Although FIGs. 5A through 5C illustrate the tubular body 115 including a support structure 122, it is contemplated that the balloon guide catheter 200 can include a tubular body 115 without the support structure 122. By not including the support structure 122, the balloon guide catheter 200 can have increased flexibility along the entire length of the tubular body 115.

As described with respect to the balloon guide catheter 100, the distal end of the tubular 115a can be modified in order to increase flexibility of the balloon guide catheter 200 at the distal end 115a, while also maintaining sufficient axial stiffness along a majority of the length of the catheter 200. The walls of the distal end 115a can have a reduced thickness as compared to the walls of the tubular body 115 extending from the distal end 115a. Alternatively or in addition to, a flexible material can be disposed at the distal end 115a of the tubular body 115 such that the flexible material is disposed under the balloon 113.

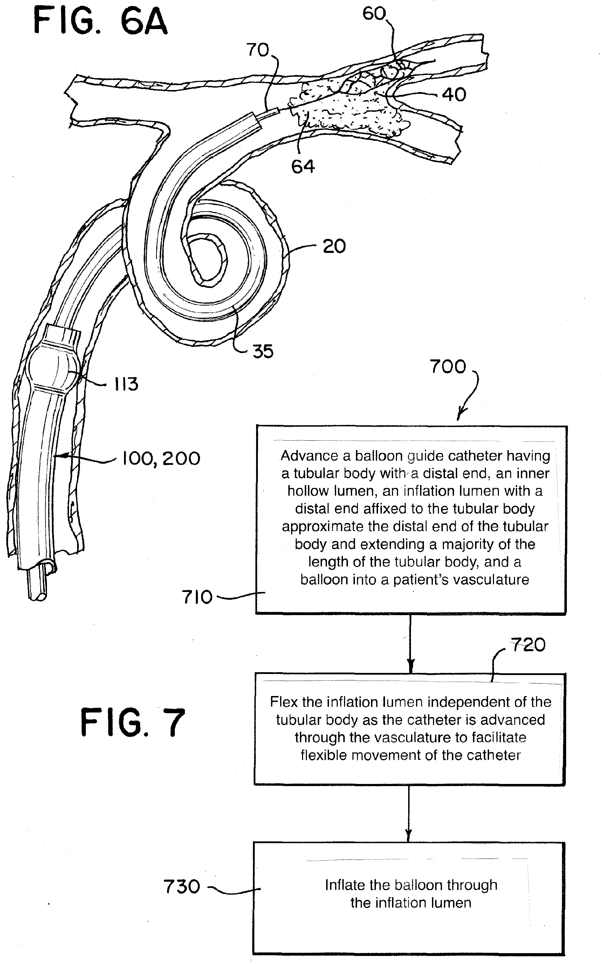

FIGs. 6A and 6B illustrate the balloon guide catheter 100, 200 used in conjunction with other mechanical thrombectomy equipment. Referring to FIG. 6A, the balloon guide catheter 100, 200 can be advanced through a target blood vessel 20 to a site proximal to an occlusive clot 40. The balloon 113 can be inflated to arrest proximal flow in the blood vessel 20. An access catheter, such as an intermediate catheter 35 or aspiration catheter can be advanced through and beyond the distal end of the balloon guide catheter 100, 200 to the target. A microcatheter 70 containing a clot retrieval device 60 can further be advanced through the intermediate catheter 35 and balloon guide catheter 100, 200 and deployed to capture the clot 40 while aspirating through one or more of the catheters. Upon capture of the clot 40, the clot retrieval device 60 can be withdrawn into the intermediate catheter 35, which can compress the structure of the clot retrieval device 60 and enhance the grip exerted on the clot during retrieval. The clot retrieval device 60, microcatheter 70, intermediate catheter 35, and clot 40 can then be withdrawn through the balloon guide catheter 100, 200 and fully removed from the patient.

As illustrated in FIG. 6B, the balloon guide catheter 100, 200 can be maneuvered through a target blood vessel, including the internal carotid artery, to a target location and the balloon 113 can be inflated. The inflated balloon 113 can block off blood proximal to the target location thereby preventing the blood from interfering with the capture of a clot. The microcatheter 70 and the clot retrieval device 60 can be advanced through the inner hollow lumen 114 and be deployed directly from the distal end 115a of the tubular body 115. The clot retrieval device 60 can include a device shaft 64 such that the clot retrieval device 60 can effectively reach and capture a clot. Aspiration can be applied through the inner hollow lumen 114 of the balloon guide catheter 100, 200 to prevent the distal migration of fragments or debris from the target site. Although the inflation lumen 117 may block a portion of the cross-section of the inner hollow lumen 114, aspiration may nevertheless be effective in some treatments and specific device configurations. As a trade-off for any potential disadvantages the position of the inflation lumen 117 poses, a potential advantage is that the distal end of the balloon guide catheter 100a can have greater flexibility as compared to a balloon guide catheter with an inflation lumen within an outer layer of the balloon guide catheter. The improved flexibility can facilitate navigating the balloon guide catheter 100a to the clot 40 and successfully retrieving the clot 40. If additional retrieval attempts are needed to clear the vessel, the microcatheter 70 and the clot retrieval device 60 can be quickly delivered back to the target site.

The clot retrieval device 60 can be any number of commercially available products. Some clot retrieval devices can compress the clot upon capture to gain a firm grip, however, this can make the clot firmer or "sticker", thereby complicating retrieval. Other clot retrieval devices can expand between the clot and the blood vessel to minimize compression while loosening the clot from the wall of the blood vessel. The clot retrieval device can be made substantially from Nitinol or other shape-memory material with sufficient elastic strain capacity such that the elastic limit would not be exceeded when the clot retrieval device is in a collapsed configuration within a microcatheter. This elastic strain capacity allows the device to be effectively "spring-loaded" within the microcatheter so that it can self-expand to engage a clot when deployed.

Placement of the balloon guide catheter 100, 200 during procedures can be aided by the addition of radiopaque markers. Such markers can include radiopaque alloying elements such as palladium, platinum, gold, and the like. For example, a radiopaque marker can be placed distal of the balloon 113 at the distal end 115a of the tubular body 115.

FIG. 7 illustrates a flow diagram outlining the method 700 of deploying a balloon guide catheter. The method 700 can include one or more of the following steps presented in no particular order. The example method 700 can include additional steps as appreciated and understood by a person skilled in the pertinent art. The example method can be performed by an example balloon guide catheter as disclosed herein, a variation thereof, or an alternative thereto as appreciated and understood by a person of ordinary skill in the art.

In step 710, a balloon guide catheter is advanced into a patient's vasculature. The balloon guide catheter can include a tubular body with a distal end, an inner hollow lumen, an inflation lumen with a distal end affixed to the tubular body approximate the distal end of the tubular body and extending a majority of the length of the tubular body, and a balloon.

In step 720, an inflation lumen independent of the tubular body can be flexed as the catheter is advanced through the vasculature to facilitate flexible movement of the catheter.

In step 730, the balloon can be inflated through the inflation lumen.

In addition to advancing the balloon guide catheter into a patient's vasculature, the method 700 can include advancing an intermediate catheter through the balloon guide catheter to the target location. Upon appropriately positioning the intermediate catheter at the target location, an occlusive thrombus can be aspirated.

The method 700 can also include advancing a microcatheter and a clot retrieval device through the balloon guide catheter to the target location. Once the microcatheter is appropriately positioned at the target location, the clot retrieval device can be deployed to capture an occlusive thrombus. The captured thrombus can then be retrieved with aspiration.

The method 700 can also include advancing an intermediate catheter, a microcatheter, and clot retrieval device through the balloon guide catheter to the target location. The clot retrieval device can be deployed to capture an occlusive thrombus. The captured thrombus can then be retrieved with aspiration.

The descriptions contained herein are examples of embodiments of the invention and are not intended in any way to limit the scope of the invention. While particular examples of the present invention are described, various modifications to devices and methods can be made without departing from the scope and spirit of the invention. For example, while the examples described herein refer to particular components, the invention includes other examples utilizing various combinations of components to achieve a described functionality, utilizing alternative materials to achieve a described functionality, combining components from the various examples, combining components from the various example with known components, etc. The invention contemplates substitutions of component parts illustrated herein with other well-known and commercially-available products. To those having ordinary skill in the art to which this invention relates, these modifications are often apparent and are intended to be within the scope of the claims which follow.

Aspects of the invention:
1. A method for deploying a balloon guide catheter, the method comprising:
   advancing a balloon guide catheter having a tubular body with a distal end, an inner hollow lumen, an inflation lumen with a distal end affixed to the tubular body approximate the distal end of the tubular body and extending a majority of a length of the tubular body, and a balloon into a patient's vasculature;
   flexing the inflation lumen independent of the tubular body as the catheter is advanced through vasculature to facilitate flexible movement of the catheter; and
   inflating the balloon through the inflation lumen.
2. The method of aspect 1, further comprising the step of advancing an intermediate catheter through the balloon guide catheter to a target location and aspirating an occlusive thrombus.
3. The method of aspect 1, further comprising the step of advancing a microcatheter and clot retrieval device through the balloon guide catheter to a target location and deploying the clot retrieval device to capture an occlusive thrombus and retrieve a thrombus with aspiration.
4. The method of aspect 1, further comprising the step of advancing an intermediate catheter, microcatheter, and clot retrieval device through the balloon guide catheter to a target location and deploying the clot retrieval device to capture an occlusive thrombus and retrieve a thrombus with aspiration.

## Claims

1. A balloon guide catheter comprising:
a substantially tubular body comprising:
an inner hollow lumen; and
a tubular body distal end;
an inflation lumen comprising:
an inflation lumen distal end in connection with the tubular body approximate the tubular body distal end; and
a proximal section extending a majority of a length of the tubular body and is movable in relation to the tubular body; and
a balloon in fluid communication with the inflation lumen distal end.

2. The balloon guide catheter of claim 1, wherein the inflation lumen comprises an inflation diameter less than a lumen diameter of the inner hollow lumen.

3. The balloon guide catheter of claim 1, wherein the inflation lumen is located i) within the inner hollow lumen, or ii) external to the tubular body.

4. The balloon guide catheter of claim 1, wherein the balloon is affixed approximate the tubular body distal end.

5. The balloon guide catheter of claim 1, wherein the tubular body comprises a wall thickness that tapers from a wider thickness at a first position to a narrower thickness at a second position, the first position being in a proximal direction from the balloon and the second position being under the balloon.

6. The balloon guide catheter of claim 1, wherein the tubular body distal end includes a flexible portion disposed under the balloon, the flexible portion being more flexible than a portion of the tubular body approximate the balloon and in a proximal direction from the balloon.

7. The balloon guide catheter of claim 3 in which the inflation lumen is located within the inner hollow lumen, wherein an inflation diameter is less than a lumen diameter of the inner hollow lumen.

8. The balloon guide catheter of claim in which the inflation lumen is located external to the tubular body 3, wherein the inflation lumen is at least partially eccentric to the tubular body.

9. A catheter comprising:
a substantially tubular body having an inner hollow lumen;
an inner surface defining the inner hollow lumen extending therethrough;
a fluidic lumen extending a majority of a length of the tubular body; and
an outer sleeve surrounding the tubular body and fluidic lumen and extending a majority of the length of the tubular body, the outer sleeve being sufficient to secure the fluidic lumen to the tubular body along the majority of the length of the tubular body.

10. The catheter of claim 9, the tubular body comprising an outer surface; and
the fluidic lumen is disposed on the outer surface of the tubular body.

11. The catheter of claim 10, the fluidic lumen is disposed on a side of the outer surface.

12. The catheter of claim 9, wherein a balloon is affixed to a distal end of the tubular body and is in fluid communication with the fluidic lumen.

13. The catheter of claim 9, wherein the fluidic lumen comprises a substantially crescent-shape cross-section.

14. The catheter of claim 9, wherein the fluidic lumen comprises a polymer such as PET.

15. The catheter of claim 9 wherein the tubular body further comprises at least one reinforcing braid or coil.
